(19) Europäisches Patentamt
European Patent Office
Office européen des brevets

(11) Numéro de publication: **0 307 267 A1**

(12) **DEMANDE DE BREVET EUROPEEN**

(21) Numéro de dépôt: **88402104.9**

(22) Date de dépôt: **12.08.88**

(51) Int. Cl.[4]: **C 10 M 173/02**
A 61 F 2/28
//(C10M173/02,145:02,155:02),
C10N40:00

(30) Priorité: **17.08.87 FR 8711642**

(43) Date de publication de la demande:
**15.03.89 Bulletin 89/11**

(84) Etats contractants désignés: **BE DE GB IT NL**

(71) Demandeur: **COMMISSARIAT A L'ENERGIE ATOMIQUE**
**Etablissement de Caractère Scientifique Technique et Industriel**
**31/33, rue de la Fédération**
**F-75015 Paris (FR)**

(72) Inventeur: **Bainville, Daniel**
**39, rue de Lattre de Tassigny**
**F-93600 Aulnay S/Bois (FR)**

**Derveaux, Guy**
**6 Square Némo**
**F-95470 Fosses (FR)**

**Wartenberg, Christian**
**198 Bld. de Créteil**
**F-94100 St. Maur des Fosses (FR)**

(74) Mandataire: **Mongrédien, André et al**
**c/o BREVATOME 25, rue de Ponthieu**
**F-75008 Paris (FR)**

(54) **Liquide de lubrification et d'environnement utilisable pour tester des prothèses osseuses.**

(57) L'invention a pour objet un liquide de lubrification et d'environnement utilisable pour tester des prothèses osseuses. Ce liquide est constitué par une solution dans un solvant d'au moins un copolymère polysiloxane-polyéther, et d'au moins un agent tensioactif capable de stabiliser la solution de copolymère à une température de 37°C.

L'agent tensioactif peut être un ester partiel d'acide polycarboxylique.

EP 0 307 267 A1

**Description**

## LIQUIDE DE LUBRIFICATION ET D'ENVIRONNEMENT UTILISABLE POUR TESTER DES PROTHESES OSSEUSES

La présente invention a pour objet un liquide de lubrification et d'environnement utilisable en particulier pour tester les propriétés de prothèses osseuses humaines.

Depuis quelques années, on a développé et utilisé de nombreux matériaux, en particulier des métaux comme l'acier inoxydable ou des alliages chrome-cobalt, et des résines synthétiques comme le polyéthylène, pour la réalisation de prothèses articulaires humaines.

Le développement de ces nouveaux matériaux a rendu nécessaire la réalisation d'essais de simulation artificielle sur les nouvelles prothèses afin d'appréhender en particulier les phénomènes d'usure.

Pour cette simulation, il faut reproduire au mieux les conditions d'environnement et d'agression rencontrées "in vivo". Dans le cas des prothèses articulaires, le milieu environnant est constitué par du sérum physiologique. Il est donc nécessaire d'utiliser pour les essais de simulation un liquide dont le coefficient de frottement et la viscosité à 37° C soient identiques à ceux du sérum physiologique. Par ailleurs, pour pouvoir exploiter les résultats d'essais, il faut, d'une part, que le liquide utilisé soit compatible chimiquement avec les matériaux constitutifs des prothèses et de l'appareillage de simulation et, d'autre part, que l'on puisse extraire de ce liquide les particules d'usure provenant des matériaux testés afin d'en analyser l'importance, la morphologie, la nature et l'origine.

Enfin, pour faciliter la réalisation des essais, il est important que le liquide utilisé soit stable, c'est-à-dire qu'il ne se dégrade pas en atmosphère non contrôlée pendant une durée de l'ordre de six mois, et qu'il conserve ses propriétés pendant toute la durée des essais.

Jusqu'à présent, on a utilisé comme liquide d'environnement et de lubrification dans les essais sur les prothèses osseuses, soit du sérum physiologique bovin, soit la solution de Ringer.

Le sérum physiologique bovin présente des propriétés intéressantes en ce qui concerne le coefficient de frottement et la viscosité à 37° C, mais il a l'inconvénient de perdre ses propriétés lubrifiantes rapidement en raison d'une putréfaction. On a pensé éviter cet inconvénient en lui ajoutant des antibiotiques, mais ceci n'a pas donné les résultats escomptés. Il est donc nécessaire de changer plusieurs fois le liquide d'environnement au cours des essais, ce qui n'est pas souhaitable.

La solution de Ringer qui est une solution de chlorure de sodium, d'hydrogénocarbonate de sodium, de chlorure de calcium et de chlorure de potassium, ne présente pas non plus toutes les propriétés requises car elle est instable chimiquement ; de plus, elle conduit à une abrasion importante des prothèses testées, ce qui ne correspond pas à la réalité.

Aussi, des recherches ont été entreprises pour trouver un liquide de lubrification et d'environnement se rapprochant mieux de la réalité physiologique, conservant ses propriétés pendant toute la durée des essais et restant stable pendant plusieurs mois.

La présente invention a précisément pour objet un liquide de lubrification et d'environnement utilisable pour tester des prothèses osseuses, qui présentent ces qualités.

Le liquide, selon l'invention, de lubrification et d'environnement utilisable pour tester des prothèses osseuses, est constitué par une solution dans un solvant d'au moins un copolymère polysiloxane-polyéther et d'un agent tensioactif capable de stabiliser la solution de copolymère à 37°C± 3°C.

Dans un tel liquide, le copolymère polysiloxane-polyéther apporte par sa composante polysiloxane, des propriétés de viscosité et de frottement identiques à celles du sérum physiologique fraîchement préparé. La partie polyéther de ce copolymère donne un caractère hydrophile marqué ainsi que des propriétés intéressantes de solubilité dans divers solvants polaires tels que l'eau, les alcools et les solutions hydroalcooliques.

A titre d'exemple, le copolymère polysiloxane-polyéther peut avantageusement répondre à la formule suivante :

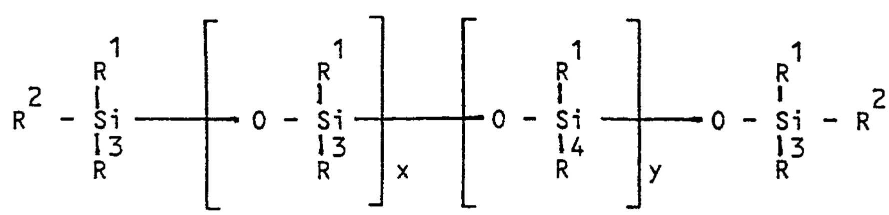

dans laquelle $R^1$, $R^2$ et $R^3$ qui peuvent être identiques ou différents, sont des radicaux alkyle ayant de 1 à 4 atomes de carbone, $R^4$ est un radical dérivé d'un copolymère statistique d'oxydes d'oléfines, par exemple d'oxyde d'éthylène et d'oxyde de propylène, et x et y sont des nombres entiers allant de 1 à 20 avec $y > x$.

Généralement, les radicaux $R^1$ , $R^2$ et $R^3$ sont des radicaux méthyle.

Le radical $R^4$ peut répondre à la formule:

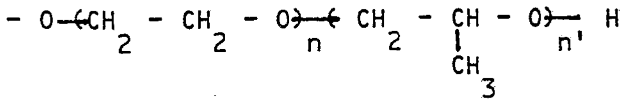

dans laquelle n et n′ sont des nombres entiers allant de 10 à 30, généralement n et n′ sont voisins.

A titre d'exemple de copolymère polysiloxane-polyéther répondant à la formule précitée, on peut citer le produit vendu sous le nom Rhodorsil 70646 par Rhône Poulenc, qui est un liquide limpide, incolore à jaune paille, ayant les caractéristiques suivantes :

- densité à 25°C : environ 1,04,
- viscosité à 25°C : 1500 $mm^2/s$,
- tension superficielle à 25°C : 20,5 mN/m.

Dans le liquide de l'invention, la présence d'un agent tensioactif a pour but de stabiliser le polymère en solution à des températures supérieures à la température ambiante.

En effet, le liquide de l'invention est destiné à la réalisation d'essais sur des prothèses articulaires, qui sont généralement effectués à la température du corps humain. Aussi, il est important que la solution de copolymère polysiloxane-polyéther reste stable à une température de 37°C.

Or, il se trouve qu'en solution aqueuse, certains copolymères polysiloxane-polyéther présentent une inversion de solubilité à ces températures.

Selon l'invention, on retarde ce phénomène de précipitation en ajoutant à la solution un agent tensioactif qui stabilise le copolymère en solution aqueuse dans la gamme de températures intéressante (autour de 37°C). Ceci permet de multiplier le nombre de cycles et la durée des essais.

De plus, le mélange de l'invention est non agressif vis-à-vis des matériaux connexes (alliages métalliques, polyéthylène, soufflets silicone).

Cet agent tensioactif peut être un ester partiel d'acide polycarboxylique ramifié comportant des radicaux hydrophiles et des radicaux hydrophobes.

Cet ester peut présenter en particulier la structure suivante :

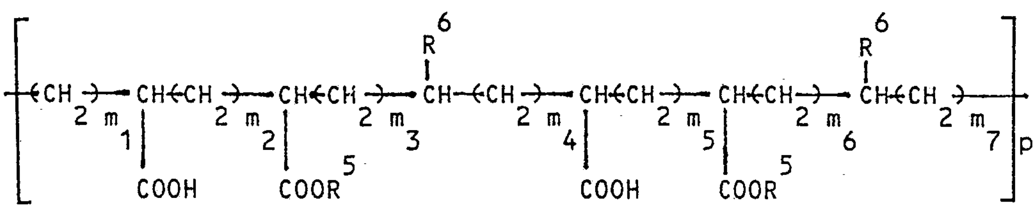

dans laquelle $m_1$, $m_2$, $m_3$, $m_4$, $m_5$, $m_6$ et $m_7$ sont des nombres entiers, identiques ou différents, allant de 1 à 10, $R^5$ est un radical hydrocarboné hydrophile ayant de 1 à 20 atomes de carbone, par exemple un radical comportant des fonctions éther-oxyde dérivé d'un polyéther, $R^6$ est un radical hydrocarboné hydrophobe, et p est un nombre entier allant de 10 à 20.

A titre d'exemple d'ester partiel d'acide polycarboxylique ramifié susceptible d'être utilisé, on peut citer le produit vendu par la Société AKZO CHEMIE sous le nom de DAPRAL GE202, qui est un liquide limpide, visqueux et jaunâtre, ayant les caractéristiques suivantes :

- poids moléculaire moyen : voisin de 20 000
- pH de sa solution à 2,5% dans l'eau : 3,7,
- viscosité à 20°C : 1200 $mm^2/s$,
- tension superficielle de la solution à 1% dans l'eau : 43 mN/m.

Cet ester est également soluble dans l'eau et dans les solvants alcooliques et hydroalcooliques.

Aussi, selon l'invention, on peut utiliser comme solvant du copolymère polysiloxane-polyéther, de l'eau, un alcool tel que le méthanol, l'éthanol, l'isopropanol ou le butanol, une cétone telle que l'acétone ou la méthyl-éthyl-cétone ou un solvant hydroalcoolique.

De préférence, on utilise l'eau car celle-ci est plus facile à manipuler ; elle est de plus économique, non toxique et chimiquement compatible avec les matériaux de prothèse et les matériaux utilisés dans les dispositifs d'essai des prothèses, et l'on peut extraire facilement d'une solution aqueuse, les particules d'usure.

Généralement, pour obtenir les propriétés souhaitées, la solution aqueuse contient de 0,1 à 1% en poids de copolymère polysiloxane-polyéther et de 0,01 à 0,1% en poids d'agent tensioactif.

Les teneurs indiquées ci-dessus en copolymère et en agent tensioactif permettent d'obtenir des formulations ayant sensiblement les caractéristiques du sérum physiologique humain tout en présentant une bonne stabilité et une bonne compatibilité chimique avec de nombreux matériaux.

Selon l'invention, on peut aussi utiliser comme liquide de lubrification et d'environnement pour tester les propriétés de prothèses humaines, une solution comprenant uniquement un copolymère polyéther-polysiloxane, lorsque le copolymère choisi reste stable en solution à 37°C.

Selon l'invention, on peut encore ajouter d'autres additifs à la solution de copolymère, ou de copolymère et d'agent tensioactif, en vue d'adapter d'autres propriétés de la solution. Ces additifs peuvent être par exemple des colloïdes, des dérivés cellulosiques, des colorants.

D'autres caractéristiques et avantages de l'invention apparaîtront mieux à la lecture de l'exemple suivant donné bien entendu à titre illustratif et non limitatif en référence au dessin annexé qui représente de façon schématique une machine pour l'essai de prothèses articulaires humaines.

Dans l'exemple suivant, on a préparé une solution aqueuse comportant :

- 0,5 % en poids du copolymère polysiloxane-polyéther commercialisé par Rhône Poulenc sous le nom de Rhodorsil 70646, et
- 0,05 % en poids d'agent tensioactif constitué par le produit vendu sous le nom de DAPRAL GE202 par la Société AKZO CHEMIE.

On a ensuite déterminé les propriétés de viscosité, de vieillissement, de compatibilité chimique et de frottement de la solution obtenue.

La viscosité a été déterminée à 25°C en utilisant l'appareillage constitué par le système cône-plan Contraves Rheomat 30.

La viscosité à 25°C était de 0,87 $mm^2/s$. A titre de comparaison, le sérum physiologique bovin présente dans les mêmes conditions une viscosité de 1,46 $mm^2/s$.

Pour tester la stabilité au stockage du liquide de l'invention, on a soumis celui-ci à des études préliminaires de vieillissement réalisées à 37°C pendant 5 semaines, et à 20°C pendant trois mois.

A la fin de ces périodes de vieillissement, le liquide de l'invention ne présentait aucune modification de ses propriétés physicochimiques et de lubrification.

Pour tester la compatibilité chimique du liquide de l'invention avec les matériaux des prothèses et des simulateurs d'articulations, on a effectué un test de compatibilité qui consiste à mettre en contact le liquide de l'invention avec un soufflet en silicone destiné à des essais sur prothèse, pendant six mois. Après ces six mois, on pèse le soufflet et on n'observe aucune perte et aucun gain de poids. De même, on n'observe aucun phénomène de gonflement.

Pour tester les propriétés de frottement du liquide de l'invention, on l'a utilisé comme liquide d'environnement et de lubrification dans une machine de compression-torsion qui permet d'imposer à une pièce une force d'appui selon un axe, et simultanément une rotation contrôlée autour de cet axe. La machine utilisée est représentée de façon schématique sur la figure unique annexée.

Dans cette machine, une des pièces en frottement est constituée par une couronne 1 usinée dans un disque de polyéthylène haute densité qui a été lui-même découpé dans une barre, l'autre pièce est constituée par un disque métallique de frottement 3 dont la face usinée est en contact avec la couronne 1. Ce disque est réalisé en acier inoxydable Z2CN18-10. Le rayon moyen de la couronne 1 est de 46 mm et sa largeur de 8 mm. Une pièce 5 du dispositif est vissée sur une tête de force 6 et une pièce 7 est fixée sur un vérin 8. Le disque de polyéthylène 1 est bridé sur la pièce 7 à l'aide d'une couronne métallique de fixation 9 et le disque de frottement métallique 3 est emboîté sur la pièce 11 puis mis en contact avec le disque de polyéthylène 1 pour les essais de frottement. Le liquide de l'invention 12 est répandu sur le disque de polyéthylène et retenu par la couronne 9. Ce vérin hydraulique 8 permet d'appliquer l'ensemble 7-11 contre la pièce 5 par l'intermédiaire de la bille 13.

En fonctionnement, le disque en polyéthylène est entraîné en rotation tandis que le disque en acier est maintenu par un capteur qui permet de mesurer la force d'appui et le couple transmis.

Les essais ont été réalisés à 37°C en appliquant une pression d'appui de 1,24 MPa qui correspond à une force de 100daN portant sur une rotule d'un diamètre de 32 mm, et en imposant une rotation alternée, selon une amplitude à variation sinusoïdale en fonction du temps, avec une période de 5s et une amplitude maximale angulaire de $\pm 10°$ (0,174 radian). Cela correspond, au moment du passage au maximum, à une vitesse linéaire relative des surfaces en contact d'environ 10 mm/s.

Les valeurs du coefficient de frottement ont été calculées selon le rapport F appui/F tangentielle, et relevées au moment du passage à vitesse maximale, dans les premiers cycles effectués. Dans ces conditions, le coefficient de frottement moyen lorsqu'on utilise le liquide de l'invention, est de 0,089 $\pm$ 0,005.

Lorsque l'on réalise le même essai en utilisant du sérum physiologique bovin fraîchement préparé au lieu du liquide de l'invention, le coefficient de frottement moyen est de 0,092 $\pm$ 0,005.

Ainsi, le liquide de l'invention a des propriétés de frottement à 37°C identiques à celles du sérum physiologique bovin. Par ailleurs, si l'on poursuit les essais pendant plusieurs heures, par exemple jusqu'à 5 heures, on n'obtient pas de variations sensibles du coefficient de frottement du liquide de l'invention.

Ainsi, le liquide de l'invention conserve ses propriétés pendant toute la durée de l'essai.

**Revendications**

1. Liquide de lubrification et d'environnement utilisable pour tester des prothèses osseuses, caractérisé en ce qu'il est constitué par une solution dans un solvant d'au moins un copolymère polysiloxane-polyéther, et d'au moins un agent tensioactif capable de stabiliser la solution de copolymère à une température de 37° C ± 3° C.

2. Liquide selon la revendication 1, caractérisé en ce que le copolymère polysiloxane-polyéther répond à la formule suivante :

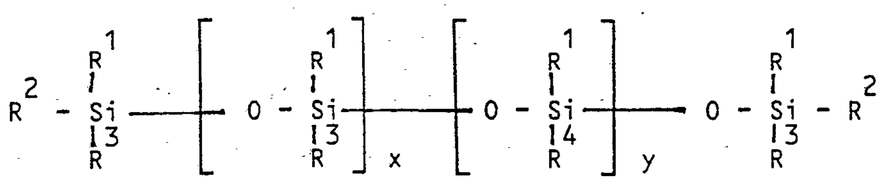

dans laquelle $R^1$, $R^2$ et $R^3$ qui peuvent être identiques ou différents, sont des radicaux alkyle ayant de 1 à 4 atomes de carbone, $R^4$ est un radical dérivé d'un copolymère statistique d'oxydes d'oléfines et x et y sont des nombres entiers allant de 1 à 20 avec $y > x$.

3. Liquide selon la revendication 2, caractérisé en ce que les oxydes d'oléfines sont l'oxyde d'éthylène et l'oxyde de propylène.

4. Liquide selon l'une quelconque des revendications 2 et 3, caractérisé en ce que $R^1$, $R^2$ et $R^3$ sont des radicaux méthyle.

5. Liquide selon l'une quelconque des revendications 2 à 4, caractérisé en ce que $R^4$ répond à la formule :

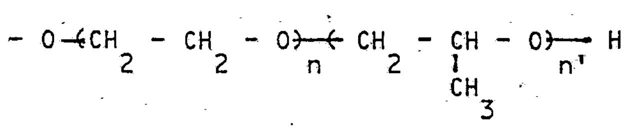

dans laquelle n et n′ sont des nombres entiers allant de 10 à 30.

6. Liquide selon l'une quelconque des revendications 1 à 5, caractérisé en ce que l'agent tensioactif est un ester partiel d'acide polycarboxylique.

7. Liquide selon la revendication 5, caractérisé en ce que l'ester partiel d'acide polycarboxylique répond à la formule:

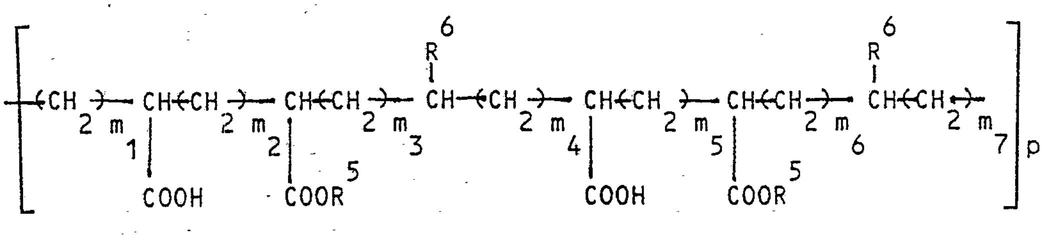

dans laquelle $m_1$, $m_2$, $m_3$, $m_4$, $m_5$, $m_6$ et $m_7$ sont des nombres entiers, identiques ou différents allant de 1 à 10, R est un radical hydrocarboné hydrophile ayant de 1 à 20 atomes de carbone, $R^6$ est un radical hydrocarboné hydrophobe et p est un nombre entier allant de 10 à 20.

8. Liquide selon la revendication 7, caractérisé en ce que le radical hydrophile $R^5$ est un radical dérivé d'un polyéther.

9. Liquide selon l'une quelconque des revendications 1 à 8, caractérisé en ce qu'il comprend de 0,1 à 1% en poids de copolymère polysiloxane-polyéther et de 0,01 à 0,1% en poids d'agent tensioactif.

10. Liquide selon l'une quelconque des revendications 1 à 9, caractérisé en ce que le solvant est l'eau.

11. Procédé pour tester les propriétés de prothèses humaines réalisées en métal et/ou en polymère organique, caractérisé en ce que les prothèses sont disposées pour l'essai dans un liquide de lubrification et d'environnement comprenant un copolymère polysiloxane-polyéther.

12. Procédé pour tester les propriétés de prothèses humaines réalisées en métal et/ou en polymère organique, caractérisé en ce que les prothèses sont disposées pour l'essai dans un liquide de lubrification et d'environnement selon l'une quelconque des revendications 1 à 10.

13. Procédé selon l'une quelconque des revendications 11 et 12, caractérisé en ce que les prothèses sont réalisées en acier inoxydable et en polyéthylène.

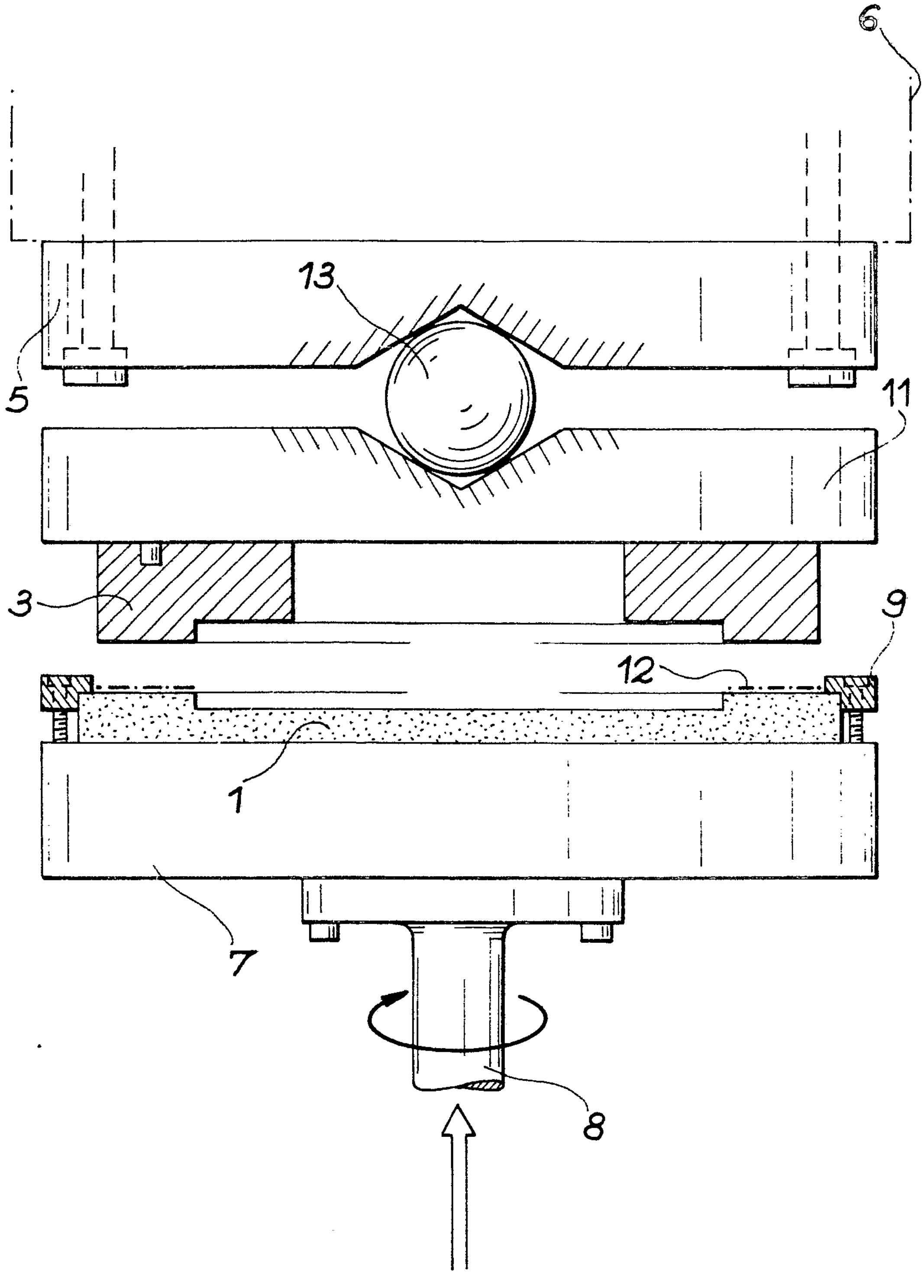

6
13
5
11
3
9
12
1
7
8

Office européen des brevets

# RAPPORT DE RECHERCHE EUROPEENNE

Numéro de la demande

EP 88 40 2104

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | PHYSICS IN MEDICINE AND BIOLOGY, vol. 23, no. 2, mars 1978, pages 253-268; A. UNSWORTH et al.: "The effects of lubrication in hip joint prostheses"<br>* Page 253, paragraphe 2 - page 254, paragraphe 3; page 252, paragraphe 3 - page 259, paragraphe 2 *<br>--- | 1,2,9-13 | C 10 M 173/02<br>A 61 F 2/28 //<br>(C 10 M 173/02<br>C 10 M 145:12<br>C 10 M 155:02 )<br>C 10 N 40:00 |
| A | ENGINEERING IN MEDICINE, vol. 10, no. 2, avril 1981, pages 89-95, MEP Ltd., Whitstable, Kent, GB; T.A. GORE et al.: "Some evidence of squeeze-film lubrication in hip prostheses"<br>* Page 89, colonne 1, paragraphe 4 - page 91, colonne 1, paragraphe 2 *<br>--- | 1,2,9-13 | |
| A | DE-A-3 321 324 (OXY-DRY CORP.)<br>* Page 7, paragraphe 2 - page 10, paragraphe 1; page 11, paragraphe 3 - page 12, paragraphe 2; pages 15-16; revendications 1,11,12 *<br>----- | 1-5,9,10 | |

| DOMAINES TECHNIQUES RECHERCHES (Int. Cl.4) |
|---|
| C 10 M<br>A 61 F |

Le présent rapport a été établi pour toutes les revendications

| Lieu de la recherche | Date d'achèvement de la recherche | Examinateur |
|---|---|---|
| LA HAYE | 24-11-1988 | HILGENGA K.J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant